# EUROPÄISCHE PATENTSCHRIFT

(11) **EP 0 605 799 B1**
(45) Veröffentlichungstag und Bekanntmachung des Hinweises auf die Patenterteilung: **14.04.1999**
(21) Anmeldenummer: 93119625.7
(22) Anmeldetag: 06.12.1993
(51) Int. Cl.: A61F 2/28, A61F 2/30, A61L 27/00

(54) **Hohlendoprothesen mit knochenwachstumsfördernder Füllung**
Hollow endoprosthesis with a filling favouring bone growth
Endoprothèse creuse avec un remplissage favorisant la croissance osseuse

(30) Priorität: 18.12.1992 DE 4242889
(43) Veröffentlichungstag der Anmeldung: 13.07.1994
(73) Patentinhaber: MERCK PATENT GmbH, 64293 Darmstadt (DE)
(72) Erfinder: Nies, Berthold, Dr., D-64372 Ober-Ramstadt (DE)

(56) Entgegenhaltungen:
- EP-A- 0 271 668
- EP-A- 0 277 678
- EP-A- 0 308 238
- EP-A- 0 309 662
- EP-A- 0 345 660
- EP-A- 0 406 856
- EP-A- 0 478 532
- EP-A- 0 493 698
- EP-A- 0 520 237
- WO-A-85/05027
- WO-A-89/04646
- CH-A- 664 686
- DE-A- 3 704 089
- DE-A- 4 130 546
- US-A- 4 936 848

## Beschreibung

Die Erfindung betrifft eine Hohlendoprothese für den Knochenersatz, die eine knochenwachstumsfördernde Füllung enthält.

Knochenimplantate sind erforderlich für den Ersatz von Knochendefekten, beispielsweise zum Schließen von Lücken nach Frakturen und Knochentumoroperationen, bei Zysten oder als Gelenkprothesen, wie insbesondere für den Hüftgelenkersatz. Knochenprothesen, die in der Regel aus körperverträglichen metallischen Werkstoffen gefertigt sind, müssen in Form und Funktion den ursprünglichen Knochen oder Knochenteilen entsprechen, die sie ersetzen sollen. Hüftendoprothesen weisen einen Schaft auf mit dem sie in den proximalen Teil des Femurs nach Entfernung von Gelenkkopf und Spongiosa eingesetzt werden. Hüftendoprothesen sind naturgemäß hohen statischen und dynamischen Belastungen ausgesetzt. Beim Prothesendesign hat sich daher das Augenmerk neben einer anatomisch angepaßten Formgebung, insbesondere sowohl auf eine anforderungsgerechte Stabilität der Prothese als solche, als auch auf ihre feste und dauerhafte Verbindung mit dem Femur zu richten. Eine ungenügende Anpassung von Festigkeit, Elastizität und Biegesteifigkeit der Prothese kann zu vorzeitigem Bruch oder Lockerung, Lösung und Ausbruch aus dem Implantationsbett führen.

Bei der Implantation kommt es auf eine kraftschlüssige Verbindung von Prothesenschaft und Knochenbett an, um eine ausreichende Primärfestigkeit zu erreichen. Je nach Prothesengestaltung kann dies durch formschlüssige Einzementierung der Prothese oder durch zementfreie Implantationstechnik, bei der Kraftschlüssigkeit gewährleistet sein muß, erfolgen. Insbesondere im zweiten Fall müssen durch entsprechende Prothesengestaltung die Voraussetzungen für einen innigen Verbund von Prothese und Knochen durch knöcherne Integration und Einheilung gegeben sein, damit die erwünschte Langzeit-Standfestigkeit der Prothese gewährleistet ist.

Neuere Entwicklungen beim Prothesendesign gehen hin zu Kohlprothesen (siehe Z. Orthop. 129, 453 (1991)). Bei Hüftprothesen wird vermehrt von der bisher üblichen massiven Ausführung abgegangen und der Prothesenschaft als Hohlschaft mit Öffnungen ausgeführt. Die Ausführung des Prothesenschaftes als Hohlkörper hat zum einen neben Material- und Gewichtseinsparung den Vorteil, daß den lokalen Erfordernissen hinsichtlich Festigkeit, Elastizität und Drehsteifigkeit besser entsprochen werden kann. Zum anderen ist es Ziel durch knöcherne Durchwachsung der Öffnungen und Einwachsung in das Schaftinnere eine dauerhafte Sekundärfestigkeit zu erreichen.

Der Neuaufbau körpereigener Knochensubstanz ist allerdings ein langwieriger Prozeß. Ohne zusätzliche künstliche Unterstützung durch knochenwachstumsfördernde Maßnahmen ist eine trabekuläre Durchwachsung der Hohlräume von Hohlendoprothesen in akzeptabelen Zeiträumen nicht zu erreichen. Die bislang beste Möglichkeit der Stimulation des knöchernen Einwachsens der Prothese ist die Auffüllung der Prothesenhohlräume mit autologem oder homologem spongiösem knochenmaterial. Autologe Spongiosa, also vom selben Individuum stammendes Material, ist in der Regel in geeigneter Art und Menge nur begrenzt verfügbar. Sofern am Implantationsort nicht direkt gewinnbar, muß es durch einen zusätzlichen operativen Eingriff an einem anderen Ort entnommen werden, was die Operation insgesamt verkompliziert, zusätzliche Schmerzen verursacht und weitere Heilungsvorgänge am Entnahmeort bedingt. Für homologes Knochenmaterial, von anderen Individuen direkt entnommen oder aus der Knochenbank, gilt sinngemäßes. Bei diesem kommen noch Probleme der Verträglichkeit aufgrund immunologischer Reaktion hinzu sowie auch die nicht völlig auszuschließende Infektionsgefahr mit Viren, wie insbesondere Hepatitis- und HIV-Viren. Weiterhin ist die Lagerung von Spendermaterial in Knochenbanken aufwendig und letztendlich zeitlich nur begrenzt.

Derartige biologische Materialien können darüberhinaus nur direkt vor oder während der Operation in die Hohlprothese appliziert werden. Eine entsprechende längerfristige Vorkonfektionierung der Prothese und Lagerung ist praktisch nicht möglich.

Die WO 85/05021 beschreibt ein künstliches Gelenksystem zur zementlosen Implantation, das ein Implantat und eine Spannvorrichtung enthält und das z.B. für künstliche Hüftgelenke, künstliche Kniegelenke etc. einsetzbar ist. Dabei ist die Spannvorrichtung bzw. insbesondere der Schaft ganz oder teilweise resorbierbar. Die Endoprothese, die mindestens eine in ein Knochenbett implantierbare, mit Öffnungen versehene Hohlstuktur aufweist, ist in der Hohlstruktur mit einer knochenwachstumsfördernden Fülhung versehen.

Die WO 89/04646 beschreibt ein Knochenersatzmaterial mit verbesserter kohäsiver und physikalischer Festigkeit, enthaltend eine nichtwäßrige Komponente wie Knochenmorphogenetische Proteinpartikel und Knochenpartikel, deren Oberfläche durch Behandlung mit 0.002-0.25 Gew.% wäss. Lösung von Glutaraldehyd aktiviert wird.

Die Beladung von Hohlendoprothesen mit körperverwandten Materialien synthetischer oder teilsynthetischer Natur, die osteoinduktive und/oder osteokonduktive Wirkung entfalten, ist möglich und auch praktikabel. Als entsprechende bioaktive

Materialien gelten Calciumverbindungen, insbesondere Calciumphosphate, wie Hydroxylapatit und Tricalciumphosphat, aber auch Calciumcarbonat, die vorzugsweise in Form von Granulaten eingesetzt werden können. Auch aus natürlichen Knochen gewonnenes Hydroxylapatit, das gegebenenfalls zur Keramik gesintert wird, ist hierfür geeignet. Alle derartigen Materialien zeigen jedoch bei weitem nicht die knochenwachstumsfördernde Wirkung von autologem und homologem Knochenmaterial.

Der Erfindung lag daher die Problemstellung zugrunde, ein Material zur Befüllung von Hohlendoprothesen zur Verfügung zu stellen, dessen knochenwachstumsfördernde Wirkung der von körpereigenem Knochenmaterial möglichst nahe kommt. Ein derartiges Knochenersatzmaterial sollte in beliebiger Menge und in zur Befüllung von Hohlprothesen geeigneter Form zur Verfügung gestellt werden können, wobei auch eine längerfristige Vorkonfektionierung und problemlose Lagerung der befüllten Prothese möglich sein sollte. Weiterhin sollte das Material wohl definiert und von reproduzierbarer und standardisierbarer Qualität sein.

Es wurde nun gefunden, daß diese Forderungen in vorzüglicher Weise von einem Knochenersatzmaterial erfüllt wird, das aus einem eine poröse Matrix bildenden Werkstoff besteht, wobei die Matrix aus gesinterter Calciumphosphatkeramik besteht und ein Polypeptid mit der biologischen Wirkung von basischem Fibroblasten-Wachstumsfaktoren (bFGF) enthält. Geeignete Materialien werden in der älteren, nicht vorveröffentlichten eigenen Anmeldung P 4121043 beschrieben.

Gegenstand der Erfindung ist somit eine Endoprothese verbesserter Sakundärfestigkeit enthaltend mindestens eine in ein Knochenbett implantierbare, mit Öffnungen versehene Hohlstruktur mit, knochenwachstumsfördernder Füllung auf Basis eines eine poröse Matrix bildenden Werkstoffs, wobei die Matrix aus gesinteter Calciumphosphatkeramik besteht und ein Polypeptid mit der biologischen Wirkung basischen Fibroblasten-Wachstumsfaktoren (bFGF) enthält.

Der mineralische Werkstoff der Matrix ist Calciumphosphat Keramik. Aus der Gruppe der Calciumphosphate sind als bevorzugt Hydroxylapatit, Tricalciumphosphat und Tetracalciumphosphat zu nennen. Derartige Calcium-Verbindungen gelten aufgrund ihrer chemischen Verwandtschaft mit der Mineralphase natürlicher Knochen als bioaktiv. Natürlicher Knochen besteht in seiner Mineralphase überwiegend aus Hydroxylapatit, einem Calciumphosphat der Summenformel Ca₅(PO₄)₃OH. Hydroxylapatit synthetischen oder organischen Ursprungs, etwa aus natürlichem Knochenmaterial, ist daher ein häufig verwendeter Rohstoff zur Herstellung von Implantatmaterialien für den Knochenersatz. Hydroxylapatit-Keramik ist im Organismus im wesentlichen nicht resorbierbar bzw. wird nur sehr langsam und über einen langen Zeitraum vom Organismus abgebaut. Das körperfremde Material bleibt über lange Zeit praktisch unverändert erhalten und die Integration in den Organismus erfolgt im wesentlichen durch Verwachsen mit vorhandenem und sich neu bildendem Knochen und Einwachsen im umgebenden Gewebe. Tricalciumphosphat ist unter bestimmten Umständen im Organismus resorbierbar. Durch Resorption vom Organismus aufgenommenes Calciumphosphat steht für den Neuaufbau von körpereigener Knochensubstanz zur Verfügung. Tetracalciumphosphat ist im wesentlichen nicht bioresorbierbar.

Ein besonders günstiges Einwachsverhalten zeigen poröse Calciumphosphat-Keramiken. Besonders bevorzugt sind hierbei Materialien basierend auf natürlichem Knochen, der durch verschiedene Behandlungen mineralisiert und durch Sinterung in ein keramisches System überführt wird, wobei die Struktur des Knochens möglichst erhalten bleiben soll. Diesen Behandlungen gemeinsam ist die Entfernung der organischen Knochenbestandteile und die anschließende Verfestigung zur Keramik durch Sinterung bei entsprechenden Temperaturen. Die Entfernung der organischen Anteile erfolgt durch chemische Lösungsvorgänge oder durch pyrolytische Verfahren. Näheres zu Knochenkeramiken und besonders günstige Verfahren zu ihrer Herstellung kann beispielsweise den Patentdokumenten DE 37 27 606, DE 39 03 695, DE 41 00 897 und DE 40 28 683 entnommen werden. Knochenkeramikwerkstoffe zeigen aufgrund ihrer ausgezeichneten Übereinstimmung mit dem Porensystem natürlichen Knochens erhebliche biologische Vorteile beim Einwachsverhalten und der Heilung im Organismus. Besonders bevorzugt ist gesinterte Spongiosa-Knochenkeramik aufgrund ihrer hochporösen, dreidimensional offenporigen Netzwerkstruktur.

Genauere Untersuchungen haben gezeigt, daß offen vorliegende mineralische Kontaktflächen in Implantatwerkstoffen aus Calciumphosphat-Keramik bevorzugt die Neubildung von mineralisierter Knochenmatrix stimulieren, wodurch sich eine festere Verwachsung des Implantats ergibt. Gefördert wird dies weiter noch bei porösen Implantaten, wo sich aufgrund der höheren Oberfläche und durch Einsprossung von neuem Knochengewebe eine besonders intensiv verzahnte und damit mechanisch stabile Verwachsung ausbildet. Bei Implantatmaterialien aus überwiegend polymeren Werkstoffen oder aus bioinerten Materialien bildet sich statt dessen zunächst bevorzugt im Kontaktbereich Bindegewebe, was zu einer nur mäßig festen Verwachsung führt.

Die porösen Matrixwerkstoffe liegen vorzugsweise in Pulver- oder Granulatform vor, können aber auch der Geometrie des Prothesenhohlraumes angepaßte Formkörper sein. Bei feinteiligen Matrixmaterialien haben diese zweckmäßigerweise eine Partikelgröße von 1-5 mm, vorzugsweise 2-4 mm. Bevorzugt sind kugelförmige Partikel, die eine leichtere und gut kompaktierbare Befüllung der Prothesenhohlräume zulassen.

Als Verbundmaterialien kommen vorzugsweise solche in Betracht, bei denen zumindest eine Komponente als poröse Matrix zur Aufnahme von Wachstumsfaktoren vorliegt. Zweckmäßig sind Werkstoffe, in denen eine poröse mineralische Matrix in Pulver- oder Granulatform vorliegt, die einen Verbund mit einem physiologisch akzeptablen polymeren Werkstoff eingeht. Verbundmaterialien dieser Art können der einschlägigen Fachliteratur entnommen werden, beispielsweise den Patentdokumenten WO 90-01342 und WO 90-01955, in denen Implantatwerkstoffe auf Basis von Calciumphosphat- bzw. Knochenkeramikpartikeln und bioresorbierbarem Polymer beschrieben sind. Typische derartige Verbundmaterialien bestehen beispielsweise aus Spongiosa-Keramik und Kollagen oder Lactid- oder Glycolidpolymeren. Je nach Art und Zusammensetzung können diese Werkstoffe als Granulat oder als mehr oder weniger plastische Massen vorliegen.

Wachstumsfaktoren, die in den erfindungsgemäßen knochenwachstumsfördernden Füllungen für Hohlendoprothesen enthalten sind, sind als solche in großer Vielfalt bekannt. Es sind dies körpereigene Peptide mit zum Teil breitem Aktivitätsspektrum in Wachstums- und Heilungsprozessen. Sie können aus biologischem Material oder durch gentechnologische Verfahren gewonnen werden. Eine umfassende Übersicht hierüber bietet beispielsweise die Monographie "Peptide Growth Factors and their Receptors I" (Editors: M.B. Sporn and A.B. Roberts) Springer Verlag Berlin, Heidelberg, New York 1990.

Für die Anwendung im Sinne der Erfindung sind basische Fibroblasten-Wachstumsfaktoren basic Fibroblast Growth Factors,bFGF) geeignet, die ebenfalls zur Klasse der körpereigenen Peptid-Wachstumsfaktoren gehören. Diese wurden ursprünglich als Substanzen in Gehirn und Hypophyse nachgewiesen und daraus isoliert und zeigten eine das Wachstum von Fibroblasten fördernde Aktivität. FGFs sind bekannt als wirksame gefäßbildende Faktoren, die u.a. für die Neovaskularisation bei der Wundheilung verantwortlich sind. Nähere Details zu FGFs einschließlich ihrer Abwandlungsprodukte, zu ihrer Isolierung bzw. Herstellung, ihrer Struktur, ihren biologischen Aktivitäten und deren Mechanismen sowie zu entsprechenden medizinischen Anwendungen können der inzwischen umfangreichen Fachliteratur entnommen werden. Eine umfassende Darstellung des aktuellen Wissens hierzu ist dem Beitrag "Fibroblast Growth Factors" von A. Baird und P. Böhlen in der vorgenannten Monographie zu entnehmen.

Zum engeren Kreis von FGFs zählen native FGFs, insbesondere bovinen und humanen Ursprungs, sowie rekombinant hergestellte FGFs. Bevorzugt sind insbesondere rekombinant hergestelltes humanes bFGF. Näheres zu rekombinant hergestellten bovinen wie humanen bFGFs kann beispielsweise den folgenden Patentdokumenten entnommen werden: EP 228 449, EP 248 819, EP 259 953, EP 275 204. Zum weiteren Kreis von FGFs zählen auch Muteine, die sich von bFGF in einem gewissen Umfang in Zahl und/oder Sequenz der Aminosäuren unterscheiden, ohne daß hiermit eine wesentliche Wirkungsveränderung verbunden ist. Der weitere Kreis von FGFs umfaßt schließlich noch verwandte Peptide mit zum Teil deutlich verschiedenen Aminosäuresequenzen mit der Wirkung von FGF sowie mit die Wirkung von FGF verstärkender Aktivität. Als Literaturhinweis seien beispielhaft die folgenden Patentdokumente angeführt: EP 148 922, EP 226 181, EP 281 822, EP 288 307, EP 319 052, EP 326 907 und WO 89-12645.

Zu FGFs im Sinne der Erfindung zählen weiterhin Derivate dieser Peptide, die mit stabilisierenden und/oder aktivitätssteigernden Agentien erhalten werden. Es sind dies insbesondere gegen Säure stabilisierte Formen von bFGF, die als stabilisierende Agentien beispielsweise Glykosaminglykane wie Heparin, Heparinfragmente, Heparansulfat und Dermatansulfat oder Glukansulfate wie Dextransulfat und Cyclodextrinsulfat enthalten. FGF-Derivate dieser Art sind beispielsweise beschrieben in EP 251 806, EP 267 015, EP 312 208, EP 345 660, EP 406 856, EP 408 146, WO 89-12464, WO 90-01941 und WO 90-03797.

Besonders bevorzugt für die Anwendung in den erfindungsgemäßen knochenwachstumsfördernden Füllungen für Endoprothesen sind Formen von rekombinant hergestelltem humanen bFGF wie sie in EP 248819 beschrieben sind.

In den erfindungsgemäßen Füllungen können die Wachstumsfaktoren in einer Konzentration von 1 µg/cm³ - 100 g/cm³ vorliegen. Die Wahl der Konzentration innerhalb des genannten Bereichs kann abhängig sein von Art und Form und der Aktivität des im Einzelfall einzusetzenden Wachstumsfaktor sowie von der Natur des im Einzelfall vorgesehenen porösen Werkstoffes und dessen ggf. inhärent vorhandener Bioaktivität.

Die Herstellung der erfindungsgemäßen Füllmaterialien durch Beladung der jeweiligen porösen Matrix mit peptidischen Wachstumsfaktoren, insbesondere mit Polypeptiden mit der Wirkung von FGF, ist an sich problemlos. Zweckmäßigerveise geht man von einer geeigneten flüssigen Präparation des Wachstumsfaktors, beispielsweise in Form einer gepufferten wäßrigen Lösung, aus und läßt diese in der vorgesehenen Dosierungsmenge in die poröse Matrix des Knochersatzmaterials vollständig einziehen. Damit, bzw. nach einer ggf. erforderlichen Trocknung, ist das Füllmaterial bereits einsetzbar oder nach den für derartige Materialien für die medizinische Anwendung erforderlichen Vorsichtsmaßnahmen lagerbar. Auf diese Weise sind poröse Formkörper, Pulver und Granulate, vorzugsweise aus Knochenkeramik, und poröse partikelförmige Komponenten für Verbundwerkstoffe mit Wachstumsfaktoren beladbar.

In sinngemäßer Weise kann die Beladung auch mit Kombinationen von verschiedenen Wachstumsfaktoren, die sich in ihrem Wirkungsspektrum ergänzen oder die Aktivitäten synergistisch beeinflussen, erfolgen. Zweckmäßig ist beispielsweise die Kombination von FGFs mit BMPs (Bone Morphogenetic Proteins).

Die erfindungsgemäßen knochenwachstumsfördernden Füllmaterialien für Hohlendoprothesen können zusätzlich zu den Wachstumsfaktoren noch mit anderen, den Knochenaufbau fördernden bzw. den Knochenabbau hemmenden Wirkstoffen beladen werden. Geeignete Wirkstoffe sind entsprechend wirksame Vitamine wie die des D-Komplexes und Hormone wie Calcitonin

Sinnvoll kann es darüberhinaus sein, die porösen Matrixmaterialien zusätzlich mit weiteren pharmazeutischen Wirkstoffen zu beladen, um hierdurch beispielsweise Infektionsrisiken zu minimieren. Geeignet sind etwa Antibiotika wie Gentamicin und Clindamycin sowie Kombinationen hiervon.

Zur Befüllung mit den knochenwachstumsfördernden Füllmaterialien sind im Prinzip alle Prothesen vorgesehen, die dem Ersatz bzw. Neuaufbau von Knochenstrukturen dienen, die zumindest teilweise in vorhandene ursprüngliche Knochenstrukturen implantiert werden und die dort eine dauerhafte Verbindung mit der körpereigenen Knochensubstanz durch Verwachsen und Einwachsen eingehen sollen. Um eine Befüllbarkeit mit den knochenwachstumsfördernden Füllmaterialien zu ermöglichen, müssen die erfindungsgemäßen Endoprothesen mindestens eine in ein Knochenbett implantierbare, mit Öffnungen versehene Hohlstruktur aufweisen, in die das Füllmaterial plaziert werden kann.

Endoprothesen für den Ersatz der verschiedensten Knochenstrukturen, die eine zur Befüllung geeignete Hohlstruktur aufweisen oder konstruktiv in diesem Sinne gestaltet werden können, sind an sich bekannt. Besonders prädestiniert, und aufgrund des medizinischen Bedarfs bevorzugt, sind Hüftgelenkendoprothesen. Diese weisen in aller Regel einen längeren, den eigentlichen Gelenkkopf tragenden Schaft auf, der in den Femur implantiert wird und der als rohrförmige und/oder mit Öffnungen versehene Hohlstruktur ausgebildet werden kann. Mit der erfindungsgemäßen knochenwachstumsfördernden Füllung versehen Hohlendoprothesen für den Hüftgelenkersatz sind eine besonders bevorzugte Realisierungsform der Erfindung.

Weitere Endoprothesen, die sinngemäß für eine Befüllung mit dem erfindungsgemäßen knochenwachstumsfördernden Füllmaterial gestaltet werden können, sind Kniegelenkprothesen, Ellbogenprothesen und Prothesen für den Wirbelkörperersatz.

Die Konfektionierung der erfindungsgemäßen Endoprothesen mit knochenwachstumsfördernder Füllung kann in verschiedener Weise und erforderlichenfalls auch in zeitlich gestufter Abfolge vorgenommen werden.

Die Befüllung der Prothesenhohlräume mit Füllmaterial in Pulver- oder Granulatform ist problemlos. Hier kann es aber angebracht sein, die Öffnungen der Prothesenhohlräume mit einer Abdeckung zu verschließen, um ein Herausfallen der Füllung bei Lagerung und Handhabung während der Operation zu vermeiden. Geeignete Abdeckmaterialien sind dünnen Netzwerke mit fliesartiger oder Gewebestruktur, die vorzugsweise aus bioabsorbierbaren Materialien wie Kollagen, Gelatine, Chitosan oder dessen Derivate oder Polyester auf Lactid- und/oder Glycolid-Basis gefertigt sind. Besonders praktikabel sind Strumpfgewebe, die einfach über die Prothese gestreift werden.

Zum gleichen Zweck kann die Füllung nachträglich mit Lösungen derartiger Biopolymere verklebt oder getränkt werden. Durch eine solche Maßnahme kann gegebenenfalls zusätzlich eine Stabilisierung oder eine Freisetzungssteuerung, der in der Füllung enthaltenen Wachstumsfaktoren und eventueller weiterer Wirkstoffe, erfolgen.

Poröse Matrixformkörper sind zweckmäßgerweise in ihrer Formgebung an die Prothesenhohlräume angepaßt, in die sie eingesetzt werden sollen.

Verbundwerkstoffe, wie insbesondere aus Spongiosa-Keramikgranulat und Lactid- bzw. Glycolidpolymeren zusammengesetzte Materialien, können so gestaltet werden, daß sie bei Raumtemperatur oder leicht erhöhter Temperatur plastisch verformbar sind. Mit derartigen plastischen Massen lassen sich Hohlendoprothesen durch einfaches Einpressen in die entsprechenden Hohlräume haltbar befüllen.

Die Befüllung der Prothesen kann entweder mit bereits mit Wachstumsfaktor beladenem Matrixmaterial erfolgen oder die Beladung der porösen Matrix wird erst nach der Befüllung der Prothese vorgenommen.

In einer möglichen Ausführungsform liegt die erfindungsgemäße Endoprothese komplett und mit wachstumsfaktorhaltiger Befüllung implantationsfertig vor. Ihre Vorzüge sind eine einfache, schnelle Handhabung und eine entsprechend kurze Operationszeit.

In einer bevorzugten Ausführungsform liegt die erfindungsgemäße Endoprothese in Form eines gebrauchsfertigen Implantationssets aus zwei oder mehr getrennten Komponenten vor, worin eine Komponente ein Prothesenformkörper ist, der die poröse Matrix enthält und eine andere Komponente eine flüssige Präparation des Polypeptids beinhaltet. Eine derartige Ausführungsform ist besonders zweckmäßig, um mögliche Stabilitätsprobleme, die bei einer Langzeitlagerung von bereits fertig konfektionierten erfindungsgemäßen Endoprothesen auftreten könnten, wirksam zu begegnen. So wurde beispielsweise in der Fachliteratur berichtet, daß Calciumionen, die ja in den hier bevorzugten Werkstoffen vorliegen, einen destabilisierenden Einfluß auf FGF ausüben können. Die Anwendung der erfindungsgemäßen Endoprothesen in Form eines derartigen Implantationssets erfolgt in der Weise, daß man kurz vor oder während des chirurgischen Eingriffs für die Implantation die poröse Matrix der Prothesenfüllung mit der den Wachstumsfaktor enthaltenden Lösung in der vorbeschriebenen Weise belädt. Hierbei ist es zweckmäßig, das Volumen der den Wachstumsfaktor enthaltenden flüssigen Präparation möglichst exakt auf das Aufnahmevermögen der porösen Matrix abzustimmen, damit eine vollständige und gleichmäßige Beladung gewährleistet ist.

Weiterhin ist eine getrennte Packung von Prothesenkörper, porösem Füllmaterial und der den Wachstumsfaktor und/oder weitere Wirkstoffe enthaltenden flüssigen Präparation in Form eines Implantationssets in Betracht zu ziehen. Auch hierbei sollten das Volumen der Prothesenhohlräume, Menge und Aufnahmevermögen des Füllmaterials und das Volumen der flüssigen Komponente aufeinander abgestimmt sein.

Die erfindungsgemäßen Hohlendoprothesen mit knochenwachstumsfördernder Füllung besitzen eine Reihe von Vorteilen, die wertvolle Verbesserungen darstellen.

Es hat sich gezeigt, daß die porösen Füllmaterialien im wesentlichen unabhängig von der Art des Werkstoffes, durch die Beladung mit Wachstumsfaktoren nach der Implantation im Kontaktbereich und, je nach dem ob sie aufgrund Porosität und/oder Resorption durchwachsbar sind, auch in ihrem Inneren eine erhebliche Neubildung von mineralisierter Knochenmatrix stimulieren. Dies ist in jedem Fall signifikant höher als in entsprechenden unbeladenen Prothesenfüllungen. Hierbei konnte bei mit FGF beladen porösen Prothesenfüllungen auf Basis von Calcium-Verbindungen, insbesondere Calciumphosphat-Keramiken, ein ausgeprägter synergistischer Effekt beobachtet werden. So zeigte sich in präklinischen Modellversuchen am Schwein bei mit FGF beladenen Knochenkeramik-Prothesenfüllungen zwölf Wochen nach der Implantation eine vollständige Inkorporation in den Knochen durch Ein- und Durchwachsung mit neugebildeter, überwiegend mineralisierter Knochenmatrix. Ein vergleichbares Ergebnis wurde nur bei einer Prothesenfüllung mit autologer Spongiosa erreicht, während beispielsweise bei unbeladener Knochenkeramik nur in den Kontaktbereichen zum vorliegenden Knochen eine Verwachsung durch Neubildung von Knochenmatrix festgestellt werden konnte. Es wird angenommen, daß die knochenwachstumsfördernde Wirkung von FGF und die Bioaktivität von Calcium-haltigen Matrixwerkstoffen, wie insbesondere Knochenkeramik, sich gegenseitig verstärken und so zu einer beschleunigten Einheilung und Inkorporation der Prothese führen.

Aus diesem verbesserten Einwachsverhalten resultiert eine verbesserte Sekundärfestigkeit und damit eine längere Standzeit der implantierten Prothese. Besonders bei Prothesen, die für die zementfreie Implantationstechnik vorgesehen sind, ist die vorliegende Erfindung ein erheblicher Fortschritt.

Da die knochenwachstumsfördernde Prothesenfüllung nicht wie im Falle autologer Spongiosa vor oder während der Implantation erst gewonnen werden muß, bestehen hierfür keine Mengenprobleme. Der operative Eingriff wird insgesamt vereinfacht, zeitlich verkürzt und für den Patienten komplikationsloser und erträglicher gestaltet.

Probleme, die mit der Verwendung von homologem Knochenmaterial als Prothesenfüllung auftreten können, wie Infektionen und immunologische Reaktionen, sind durch die Erfindung ausgeschlossen.

Mit der erfindungsgemäßen knochenwachstumsfördernden Prothesenfüllung steht ein wohl definierbares, reproduzierbares Material mit standardisierbarer Qualität und steuerbarer biologischer Aktivität zur Verfügung.

### Beispiel 1

### Befüllte Hüftgelenkprothese

Eine vorgefertigte handelsübliche Hüftgelenkprothese aus Titanlegierung, die einen rohrförmigen, mit Öffnungen versehenen Hohlschaft aufweist, wird vollständig mit einem Granulat (Partikelgröße 2-4 mm) aus Hydroxylapatit-Spongiosakeramik (hergestellt nach DE 40 28 683) befüllt. Auf die poröse Füllung wird mittels Spritze bis zur Sättigung eine gepufferte Lösung von rekombinant hergestelltem, humanen bFGF in der Weise aufgegeben, daß die Beladung 50 µg bFGF/cm³ Prothesenfüllung beträgt.

Die Prothese wird unter sterilen Bedingungen gefriergetrocknet und steril verpackt. Sie ist dann implantationsbereit.

### Beispiel 2

### Implantationsset

Eine wie in Beispiel 1 mit Hydroxylapatit-Spongiosakeramik befüllte Hüftgelenkprothese, jedoch ohne Beladung mit bFGF, wird sterilisiert und steril verpackt.

bFGF-Lösung in Citrat-Puffer (10 mMol; pH 5,0) wird nach Zugabe von Saccharose-Lösung (9 %) gefriergetrocknet und in Ampullen abgefüllt. Dabei werden Ampullenbefüllung und Ampullenvolumen so abgestimmt, daß die spätere Beladung der Prothesenfüllung 50 µg bFGF/cm³ entspricht.

Prothesen-Packungen und bFGF-Ampullen bilden Packungseinheiten als Implantationssets.

### Konditionierung am Operationstisch

Die bFGF-Lösung wird in Citrat-Puffer (pH 5,0) rekonstituiert und anschließend auf eine sterile Spritze aufgezogen.

Nach Öffnen der Verpackung wird die bFGF-Lösung auf die poröse Prothesenfüllung aufgegeben. Das Injektionsvolumen wird so bemessen, daß die Füllung vollständig mit der bFGF-Lösung gesättigt wird. Überschüssige bFGF-Lösung wird nach ca. 1 Minute in die Spritze zurückgesaugt. Die Prothesenfüllung hält etwa so viel Lösung fest, wie seinem Porenvolumen entspricht.

Die beladene Prothese kann nun implantiert werden.

## Patentansprüche

1. Endoprothese mit verbesserter Sekundärfestigkeit, enthaltend mindestens eine in ein Knochenbett implantierbare, mit Öffnungen versehenen Hohlstruktur mit knochenwachstumsfördernder Füllung auf Basis eines eine poröse Matrix bildenden Werkstoffs, dadurch gekennzeichnet, daß die Matrix aus gesinterter Calciumphosphatkeramik besteht und ein Polypeptid mit der biologischen Wirkung von basischem Fibroblasten-Wachstumsfaktor (bFGF) enthält.

2. Endoprothese nach Anspruch 1, dadurch gekennzeichnet, daß sie in der porösen Matrix 1 - 100 µg/cm³ an basischem Fibroblasten-Wachstumsfaktor enthält.

3. Endoprothese gemäß Anspruch 1, dadurch gekennzeichnet, daß sie in Form eines gebrauchsfertigen Implantationssets aus zwei oder mehr getrennten Komponenten vorliegt, dessen eine Komponente ein Prothesenformkörper ist, der die poröse Matrix enthält, und eine andere Komponente eine flüssige Präparation des Polypeptids beinhaltet.

## Claims

1. Endoprosthesis with improved secondary strength, comprising at least one hollow structure which can be implanted in a bone bed and is provided with openings, and which has a filling which promotes bone growth and is based on a material which forms a porous matrix, characterized in that the matrix consists of sintered calcium phosphate ceramic and comprises a polypeptide with the biological effect of basic fibroblast growth factor (bFGF).

2. Endoprosthesis according to Claim 1, characterized in that it contains in the porous matrix 1-100 µg/cm³ of basic fibroblast growth factor.

3. Endoprosthesis according to Claim 1, characterized in that it is in the form of an implantation kit which is ready for use and consists of two or more separate components, one component of which is a prosthesis shaped article which contains the porous matrix, and another component contains a liquid preparation of the polypeptide.

## Revendications

1. Endoprothèse présentant une résistance mécanique secondaire améliorée comportant moins une structure creuse munie d'ouvertures, implantable dans un lit osseux avec un remplissage favorisant la croissance osseuse à base d'un matériau formant une matrice poreuse, caractérisée en ce que la matrice est constituée d'une céramique à base de phosphate de calcium frittée et contient un polypeptide présentant l'activité biologique du facteur de croissance des fibroblastes basique (bFGF).

2. Endoprothèse selon la revendication 1, caractérise en ce qu'elle contient dans la matrice poreuse 1 à 100 µg/cm³ du facteur de croissance des fibroblastes basique.

3. Endoprothèse selon la revendication 1, caractérisée en ce qu'elle se présente sous là forme d'un ensemble implantable prêt à l'emploi constitué de deux ou plusieurs composants séparés, l'un des composants étant le corps moulé de la prothèse contenant la matière poreuse et l'autre composant comportant une préparation liquide de polypeptide.
